# EUROPEAN PATENT SPECIFICATION

(11) **EP 2 875 791 B1**
(45) Date of publication and mention of the grant of the patent: **25.12.2019**
(21) Application number: 14194536.0
(22) Date of filing: 24.11.2014
(51) Int. Cl.: A61B 18/14, A61B 17/00, A61B 90/00

(54) **Irrigated catheter tip with temperature sensor and optic fiber arrays**
Spülkatheterspitze mit Temperatursensor- und Glasfaserarrays
Pointe de cathéter irriguée avec capteurs de température et réseaux de fibre optique

(30) Priority: 26.11.2013 US 201314090614
(43) Date of publication of application: 27.05.2015
(73) Proprietor: Biosense Webster (Israel) Ltd., 2066717 Yokneam (IL)
(72) Inventor: Govari, Assaf, 3440001 Haifa (IL); Beeckler, Christopher Thomas, Brea, CA California 92821 (US); Hettel, Rowan Olund, Pasadena, CA California 91107 (US); Papioannou, Athanassios, Los Angeles, CA California 90066 (US)
(74) Representative: Carpmaels & Ransford LLP

(56) References cited:
- EP-A1- 1 922 991
- US-A1- 2007 287 998
- US-A1- 2008 275 428
- US-A1- 2013 204 134

## Description

### FIELD OF THE INVENTION

The present invention relates generally to invasive medical devices, and particularly to probes used in ablating tissue within the body.

### BACKGROUND

Minimally-invasive intracardiac ablation is the treatment of choice for various types of arrhythmias. To perform such treatment, the physician typically inserts a catheter through the vascular system into the heart, brings the distal end of the catheter into contact with myocardial tissue in areas of abnormal electrical activity, and then energizes one or more electrodes at or near the distal end in order to create tissue necrosis.

It has been found that cooling the area of the ablation site reduces tissue charring and thrombus formation. For this purpose, for example, Biosense Webster Inc. (Diamond Bar, Calif.) offers the ThermoCool® irrigated-tip catheter for use with its CARTO® integrated mapping and ablation system. The metal catheter tip, which is energized with radio-frequency (RF) electrical current to ablate the tissue, has a number of peripheral holes, distributed circumferentially around the tip, for irrigation of the treatment site. A pump coupled to the catheter delivers saline solution to the catheter tip, and the solution flows out through the holes during the procedure in order to cool the catheter tip and the tissue.

U.S. Patent Application Publication 2010/0030209 describes a catheter with a perforated tip, which includes an insertion tube, having a distal end for insertion into a body of a subject. A distal tip is fixed to the distal end of the insertion tube and is coupled to apply energy to tissue inside the body. The distal tip has an outer surface with a plurality of perforations through the outer surface, which are distributed circumferentially and longitudinally over the distal tip. A lumen passes through the insertion tube and is coupled to deliver a fluid to the tissue via the perforations.

Some ablation catheters include sensors for monitoring temperature during the ablation procedure. For example, U.S. Patent 5,957, 961 describes a catheter having a distal segment carrying at least one electrode extending along the segment and having a number of temperature sensors arranged along the distal segment adjacent the electrode, each providing an output indicative of temperature. The catheter is coupled to a power source, which provides RF energy to the electrode. Temperature processing circuitry is coupled to the temperature sensors and the power source, and controls power output from the power source as a function of the outputs of the temperature sensors.

As another example, U.S. Patent 6,312, 425 describes an RF ablation catheter tip electrode with multiple thermal sensors. A tip thermal sensor is located at or near the apex of the distal-end region, and one or more side thermal sensors are located near the surface of the proximal-end region. The electrode is preferably an assembly formed from a hollow dome-shaped shell with a core disposed within the shell. The side thermal sensor wires are electrically connected inside the shell and the core has a longitudinal channel for the side thermal sensor wires welded to the shell. The shell also preferably has a pocket in the apex of the shell, and the end thermal sensor wires pass through the core to the apex of the shell.

It has also been found that contact between the ablation electrode and the tissue being ablated affects the efficacy of the ablation, so that methods for detection of tissue-electrode contact have been developed. For example, U.S. Patent 6,217, 574 describes an irrigated split tip electrode catheter. A signal processor activates an RF generator to transmit a low level RF current to each electrode member of the split tip electrode. The signal processor receives signals indicative of the impedance between each electrode member and one or more surface indifferent electrodes and determines which electrode members are associated with the highest impedance. Such electrode members are stated to be those in greatest contact with the myocardium.

As another example, U.S. Patent 6,391, 024 describes a method of assessing the adequacy of contact between an ablation electrode and biological tissue. The method measures the impedance between an ablation electrode and a reference electrode at a first and second frequencies. A percentage difference between the first-frequency impedance and the second-frequency impedance is stated to provide an indication of the state of electrode/tissue contact.

As yet another example, U.S. Patent 6,730, 077 describes a cryocatheter for treatment of tissue. A signal conductor extends through the catheter to the catheter tip and connects to a thermally and electrically conductive shell or cap that applies an RF current to the region of tissue contacted by the tip. A tissue impedance path between the signal lead and a surface electrode mounted on the patient's skin is monitored to develop a quantitative measure of tissue contact at the distal tip,

US2013204134A1 discloses a property determination apparatus for determining a property of an object. Optical sensing data being indicative of an optical property of the object and ultrasound sensing data being indicative of an ultrasound property of the object are generated, and a property determination unit determines a property of the object based on at least one of the optical sensing data and the ultrasound sensing data

US2007287998A1 discloses a catheter with a multi-port tip for optical lesion evaluation.

EP1922991A1 discloses a catheter with omni-directional optical tip having isolated optical paths. The catheter tip design isolates illumination and collection paths within the tip electrode such that light for illuminating the tissue of interest (e.g., cardiac tissue or blood) is isolated within the tip electrode from light that returns from the tissue to the catheter tip, and vice versa.

### SUMMARY

The invention is defined in appended independent claim 1, preferred embodiments are described in the dependent claims.

An embodiment of the present invention provides apparatus, consisting of:
an insertion tube having a distal end configured for insertion into proximity with tissue in a body of a patient and containing a lumen having an electrical conductor for conveying electrical energy to the tissue;
a conductive cap attached to the distal end of the insertion tube and coupled electrically to the electrical conductor, wherein the conductive cap has an outer surface; and
a multiplicity of optical fibers contained within the insertion tube, each fiber terminating in proximity to the outer surface of the cap, and being configured to convey optical radiation to and from the tissue while the electrical energy is being conveyed to the tissue.

Typically the apparatus includes a plurality of temperature sensors, which are mounted within the conductive cap in thermal communication with the outer surface.

In a disclosed embodiment the outer surface is penetrated by multiple apertures, and the conductive cap defines an inner cavity in fluid communication with the lumen of the insertion tube so as to permit irrigation fluid from the lumen to flow out of the cap through the apertures.

The cap may have a side wall having a multiplicity of longitudinal bores therein, and the fiber optics are inserted into the bores. The apparatus may also have a multiplicity of windows, transparent to the optical radiation, located in the outer surface of the cap and connected to respective longitudinal bores so as to seal the bores from penetration of fluid into the bores. Typically, at least one of the windows is formed from at least one of a transparent epoxy and a glue. The apparatus may include a scattering agent mixed with at least one of the windows. Typically, at least one of the windows is formed from at least one of optical grade flat material and optical grade lensed material.

In a further disclosed embodiment a given optical fiber selected from the multiplicity of optical fibers consists of a single optical fiber. Alternatively or additionally, a given optical fiber selected from the multiplicity of optical fibers consists of an optical fiber bundle.

According to the invention the apparatus has an optical module configured to determine contact of the conductive cap with the tissue in response to measuring a first level of the optical radiation conveyed via a given optical fiber to the tissue and a second level of the optical radiation conveyed via the given optical fiber from the tissue.

In an alternative embodiment the apparatus includes an optical module configured to determine a characteristic of the tissue in response to measuring a first level of the optical radiation conveyed via a first optical fiber to the tissue and a second level of the optical radiation conveyed via a second optical fiber from the tissue. Typically, the characteristic includes a wall thickness of the tissue.

In a further alternative embodiment the apparatus includes a power generator, coupled to provide the electrical energy to the conductive cap so as to ablate the tissue, and an optical module configured to determine a change in a level of the optical radiation while the tissue is ablating.

There is further disclosed a method, including:
inserting a distal end of an insertion tube into proximity with tissue in a body of a patient;
forming a lumen, including an electrical conductor for conveying electrical energy to the tissue, within the insertion tube;
attaching a conductive cap, having an outer surface, to the distal end of the insertion tube;
coupling the conductive cap electrically to the electrical conductor; and
locating a multiplicity of optical fibers within the insertion tube, each fiber terminating in proximity to the outer surface of the cap, and being configured to convey optical radiation to and from the tissue while the electrical energy is being conveyed to the tissue.

The present invention will be more fully understood from the following detailed description of the embodiments thereof, taken together with the drawings in which:

### BRIEF DESCRIPTION OF THE DRAWING

Fig. 1 is a schematic, pictorial illustration of a system for intracardiac ablation, in accordance with an embodiment of the present invention;
Fig. 2A is a schematic, sectional view of a catheter tip, in accordance with an embodiment of the present invention;
Fig. 2B is a schematic, cross-sectional view of the catheter tip of Fig. 2A;
Fig. 3 is a schematic, sectional view of a catheter tip, in accordance with another embodiment of the present invention;
Fig. 4A is a schematic, pictorial illustration of a catheter cap, in accordance with yet another embodiment of the present invention;
Fig. 4B is a schematic end view of the catheter cap of Fig. 4A;
Fig. 4C is a schematic, sectional view of the catheter cap of Figs. 4A and 4B; and
Figs. 5A - 5E are schematic illustrations of a catheter cap, and of light paths to/from the cap, according to an alternative embodiment of the present invention.

### DETAILED DESCRIPTION OF EMBODIMENTS

Intracardiac ablation procedures are characterized by rapid temperature changes and non-uniform temperature distribution in the tissue and its vicinity. Therefore, the temperature measured by a sensor at the tip of an ablation catheter may not accurately reflect the actual, current temperature in the tissue. Furthermore, when a temperature sensor in a catheter is washed by irrigation fluid, the temperature reading will reflect the fluid temperature, which is generally far cooler than the tissue temperature outside the catheter.

Some embodiments of the present invention that are described hereinbelow provide irrigated ablation electrodes with embedded temperature sensors that provide accurate tissue temperature assessment. Such electrodes typically comprise a conductive cap, which is attached to the distal tip of the insertion tube of an invasive probe, such as a cardiac catheter. A cooling fluid flows out through an array of perforations in the electrode to irrigate the tissue under treatment.

The temperature sensors are mounted at different locations in proximity to the outer surface of the electrode. The electrode is constructed so that the sensors are in proximity to and thermal communication with the outer surface, and are thermally insulated from, rather than immersed in, the cooling fluid within the probe. The sensors thus provide multiple temperature readings that are substantially independent of the cooling fluid temperature, at different locations on the tip electrode.

Typically, the sensor that gives the highest temperature reading is the one that is in contact with the tissue being ablated, and the temperature measured by this sensor varies linearly with the actual tissue temperature. (Flow of the cooling fluid through the perforations in the electrode is generally lowest in areas that are in firm contact with the tissue, and the sensors in these areas typically give the highest temperature readings.) The reading from this hottest sensor may thus be used in particular to monitor the tissue temperature and control the applied power and duration of the ablation procedure in order to obtain the desired therapeutic result without excessive tissue damage. Alternatively or additionally, the temperature readings of the multiple sensors can be combined and interpolated to give a map of temperature over the area of the catheter tip.

Some embodiments of the present invention incorporate a multiplicity of optical fibers terminating in proximity to the outer surface of the conductive cap. The optical fibers may transmit optical radiation to the tissue being ablated, and also receive returning optical radiation from the tissue. Measurements of levels of returning radiation at all or some of the fiber optics enable embodiments of the present invention to determine if the conductive cap is in contact with the tissue, as well as to characterize the tissue being irradiated by the radiation.

Although the disclosed embodiments relate specifically to intracardiac catheters and ablation procedures, the principles of the present invention may similarly be applied, *mutatis mutandis*, to probes of other types, for use in substantially any sort of invasive thermal treatment.

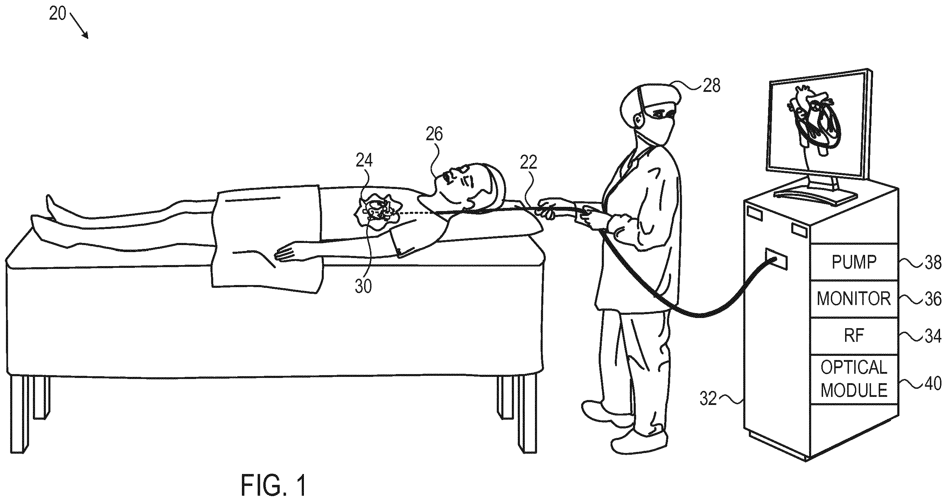

Fig. 1 is a schematic pictorial illustration of a system 20 for cardiac ablation treatment, in accordance with an embodiment of the present invention. An operator 28 (such as an interventional cardiologist) inserts a catheter 22 via the vascular system of a patient 26 into a chamber of the patient's heart 24. For example, to treat atrial fibrillation, the operator may advance the catheter into the left atrium and bring a distal end 30 of the catheter into contact with myocardial tissue that is to be monitored and/or ablated.

Catheter 22 is connected at its proximal end to a console 32, which is controlled by operator 28 to apply and monitor the desired treatment. Console 32 comprises an RF energy generator 34, which supplies electrical power via catheter 22 to distal end 30 in order to ablate the target tissue. Monitoring circuitry 36 tracks the temperature of the tissue at distal end 30 by processing the outputs of temperature sensors in the distal end, as described below. An irrigation pump 38 supplies a cooling fluid, such as saline solution, through catheter 22 to irrigate distal end 30. In addition, an optical module 40 provides optical radiation, typically from, but not limited to, a laser, an incandescent lamp, an arc lamp, or a light emitting diode (LED), for transmission from distal end 30 to the target tissue. The module receives and analyzes optical radiation returning from the target tissue and acquired at the distal end, as described below.

On the basis of information provided by monitoring circuitry 36 and optics module 40, console 32 may control the power applied by RF energy generator 34 and/or the flow of fluid provided by pump 38, either automatically or in response to inputs by operator 28.

System 20 may be based on the above-mentioned CARTO system, for example, which provides extensive facilities to support navigation and control of catheter 22. These system facilities, however, including details of the monitoring and control functions of monitoring circuitry 36 and console 32 generally, are beyond the scope of the present patent application.

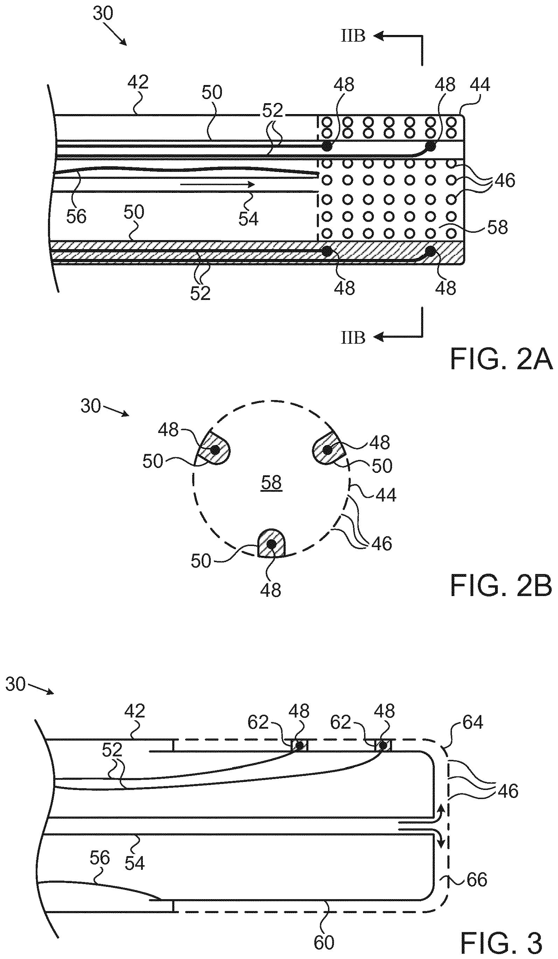

Figs. 2A and 2B schematically illustrate distal end 30 of catheter 22, in accordance with an embodiment of the present invention. Fig. 2A is a sectional view along the length of the catheter, while Fig. 2B is a cross-sectional view along the cut IIB-IIB that is marked in Fig. 2A. An insertion tube 42 extends along the length of the catheter and is connected at its distal end to a conductive cap 44. Typically, insertion tube 42 comprises a flexible, biocompatible polymer, while cap 44 comprises a biocompatible metal suitable to serve as an ablation electrode, such as gold or platinum, for example. Cap 44 is perforated by an array of irrigation apertures 46, which is open from the outer surface of the cap into an inner cavity 58 within the cap. For typical intracardiac ablation applications, the diameter of cap 44 may be about 2.5 mm, with a wall thickness of about 0.2 mm and apertures 46 of diameter 0.1-0.2 mm. The above dimensions and materials are described by way of example, however, and other suitable materials, with features of larger or smaller dimensions, may similarly be used.

Cavity 58 is in fluid communication with a lumen 54, which runs through the length of insertion tube 42. Lumen 54 is coupled at its proximal end to irrigation pump 38, and thus conveys irrigation fluid to cavity 58, from which the fluid flows out through apertures 46. An electrical conductor 56 conveys electrical energy from RF generator 34, through insertion tube 42, to cap 44, and thus energizes the cap to ablate myocardial tissue with which the cap is in contact. During ablation, the fluid flowing out through apertures 46 irrigates the tissue under treatment.

Temperature sensors 48 are mounted within conductive cap 44 at locations that are arrayed around the distal tip of the catheter, both axially and circumferentially. In this example, cap 44 contains six sensors, with one group in a distal location, close to the tip, and the other group in a slightly more proximal location. This distribution is shown only by way of example, however, and greater or smaller numbers of sensors may be mounted in any suitable locations within the cap. Sensors 48 may comprise thermocouples, thermistors, or any other suitable type of miniature temperature sensor. These sensors are connected by leads 52 running through the length of insertion tube 42 to provide temperature signals to monitoring circuitry 36.

Temperature sensors 48 are mounted within ribs 50 inside cap 44. The ribs are typically an integral part of cap 44 and may be made from the same material as the outer surface of the cap or from some other suitable type of metal, which is physically and thermally bonded to the cap. The diameter of the ribs may be a few tenths of a millimeter in the present example. The integral construction of ribs 50 with cap 44 causes sensors 48 to be in thermal communication with the outer surface of the cap, i.e., the temperature inside ribs 50 closely tracks the temperature of the outer surface. The ribs are thick enough to thermally insulate these sensors from the irrigation fluid in cavity 58. As a result, temperature sensors 48 measure the true temperature of the outer surface of cap 44, which most accurately reflects the temperature of the tissue with which the cap is in contact.

Typically, distal end 30 contains other functional components, which are outside the scope of the present disclosure and are therefore omitted for the sake of simplicity. For example, the distal end of the catheter may contain steering wires, as well as sensors of other types, such as a position sensor and/or a contact force sensor. A catheter containing sensors of these sorts is described, for example, in U.S. Patent Application Publication 2009/0138007.

Fig. 3 is a schematic sectional view of distal end 30, in accordance with another embodiment of the present invention. Elements of this embodiment that are similar to corresponding elements in the embodiment of Figs. 2A and 2B are marked with the same indicator numbers. In the embodiment of Fig. 3, a conductive, perforated cap 64, attached to the distal end of insertion tube 42, is designed to have very low thermal capacity, and sensors 48 are held in contact with cap 64. As a result of this configuration, the temperature of cap 64 more closely track changes in the actual tissue temperature, and sensors 48 more closely track the temperature of the outer surface of cap 64. Sensors 48 thus provide a more accurate, timely indication of changes in the temperature of the tissue with which cap 64 is in contact.

As illustrated in Fig. 3, cap 64 contains an inner wall 60, which is not perforated, in close proximity and parallel to the cap. Lumen 54 supplies irrigation fluid to a cavity 66 that is formed between cap 64 and wall 60, and the irrigation fluid exits this cavity through apertures 46 in cap 64. Typically, cap 64 and wall comprise thin shells of metallic material and are held apart by small metallic spacers 62, around which the fluid is able to flow within cavity 66. These spacers may be distributed within cap in any suitable arrangement, for example in pairs (like the pair shown in Fig. 3) of axially-spaced sensors at different circumferential locations. Spacers 62 also hold temperature sensors 48 in thermal communication with the outer surface of cap 64, while insulating the sensors from the surrounding irrigation fluid in cavity 66. Even without the insulating effect of spacers 62, the effect of the irrigation fluid temperature on sensors 48 in this embodiment is minimal due to the small volume of cavity 66 (relative to cavity 58 in the preceding embodiment, for example).

In a configuration suitable for intracardiac ablation, cap 64 has an outer diameter of about 2.5 mm and a similar length. The thickness of both cap 64 and wall 60 is about 100 µm, while apertures 46 have a diameter in the range of 25-100 µm. Although cap 64 and wall 60 are very thin, the mechanical integrity of the entire structure is maintained by connecting the cap and wall together with spacers 62.

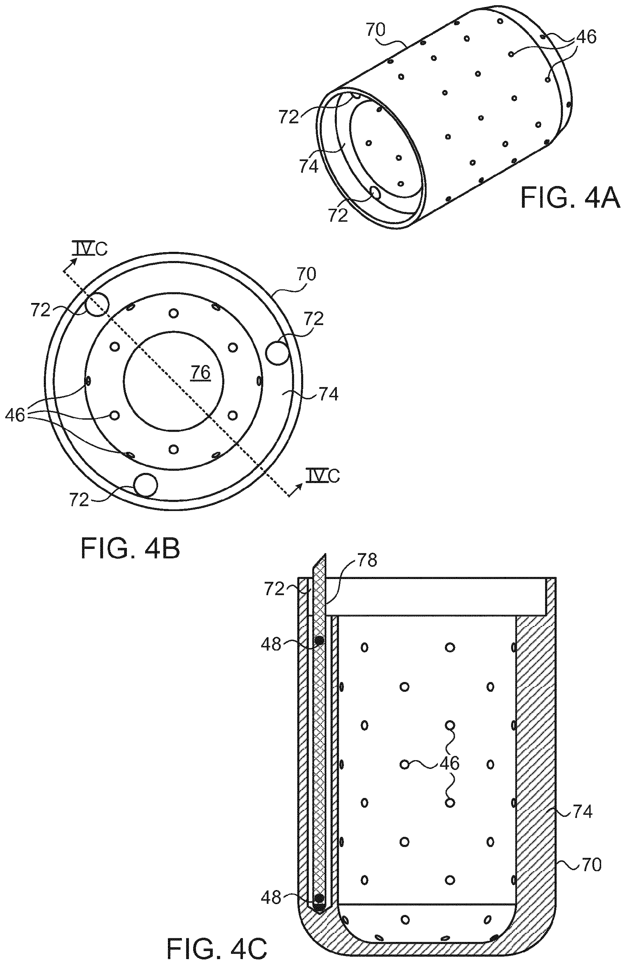

Figs. 4A-4C schematically illustrate a catheter cap 70, in accordance with yet another embodiment of the present invention. Cap 70 may be used at distal end 30 of catheter 22 in place of the caps shown in the preceding embodiments. Fig. 4A is a schematic, pictorial illustration of cap 70, while Fig. 4B is a schematic end view showing the interior of the cap, and Fig. 4C is a sectional view taken along the line IVC-IVC in Fig. 4B.

Cap 70 comprises a side wall 74 that is relatively thick, on the order of 0.4 mm thick, in order to provide the desired thermal insulation between temperature sensors 48 and the irrigation fluid inside a central cavity 76 of the tip. As in the preceding embodiments, the irrigation fluid exits cavity 76 through apertures 46. Sensors 48 are mounted in hollow tubes 78, which are filled with a suitable glue, such as epoxy and fitted into longitudinal bores 72 in side wall 74. Tubes 78 may comprise a suitable plastic material, such as polyimide, and may be held in place by a suitable glue, such as epoxy. This arrangement provides an array of six sensors 48 as in the preceding embodiments, with possible advantages of greater ease of manufacture and durability.

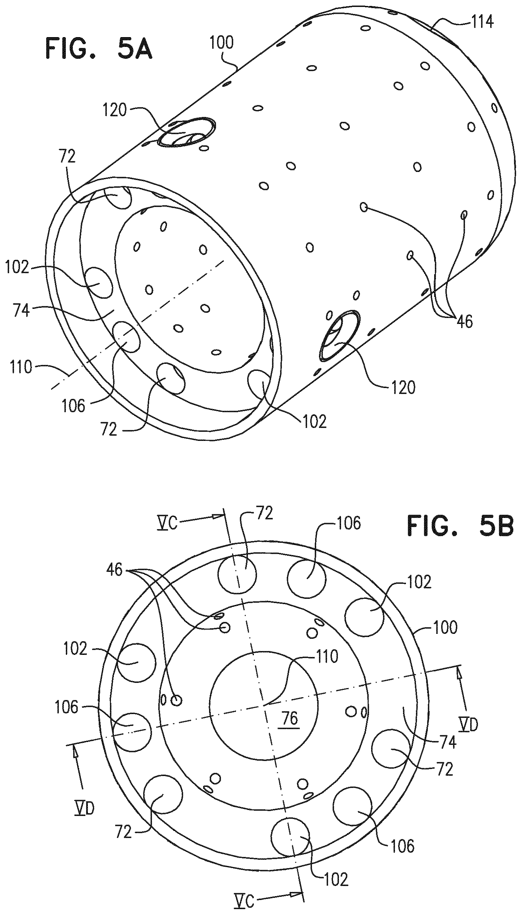

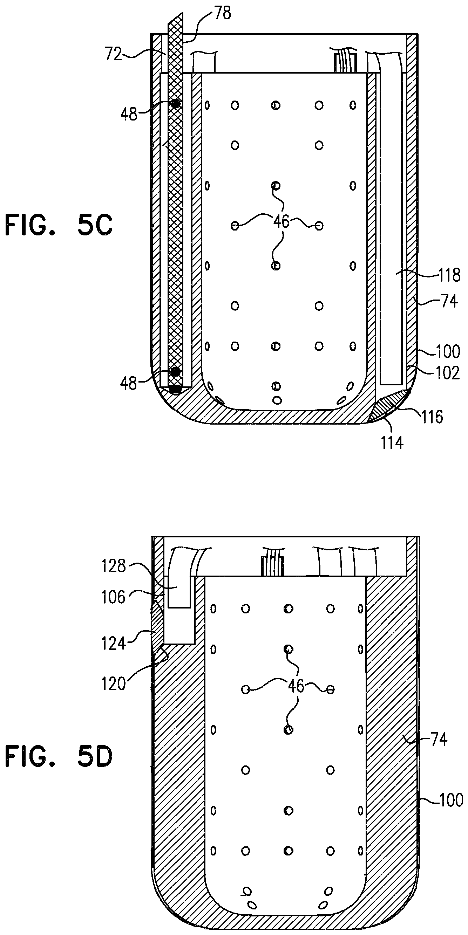

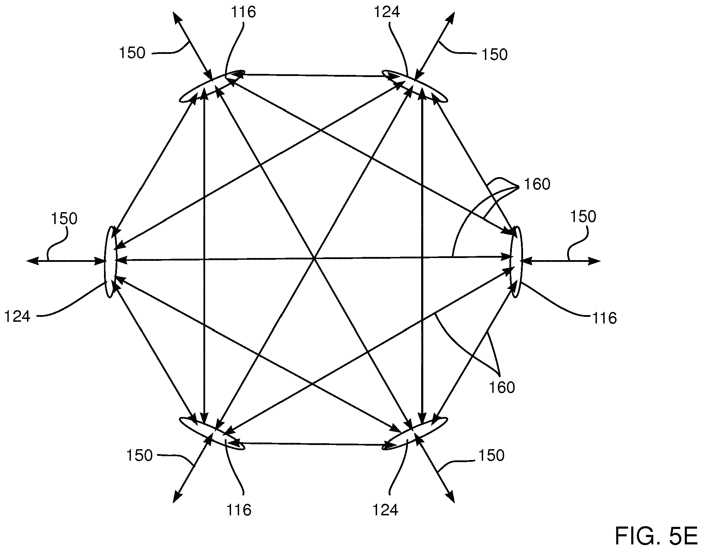

Figs. 5A-5D schematically illustrate a catheter cap 100, and Fig. 5E schematically illustrates paths taken by light to/from windows in the cap, in accordance with an alternative embodiment of the present invention. Apart from the differences described below, the operation of cap 100 is generally similar to that of cap 70 (Figs. 4A, 4B, and 4C), and elements indicated by the same reference numerals in both caps 70 and 100 are generally similar in construction and in operation. Fig. 5A is a schematic, perspective illustration of cap 100, Fig. 5B is a schematic end view showing the interior of the cap, Fig. 5C is a schematic sectional view taken along the line VC-VC in Fig. 5B, and Fig. 5D is a schematic sectional view taken along the line VD-VD in Fig. 5B.

In addition to the three longitudinal bores 72 described above for cap 70, wherein sensors 48 mounted in tubes 78 are fitted, in cap 100 three through longitudinal bores 102, and three blind longitudinal bores 106 are formed in side wall 74. As is illustrated in Fig. 5B, the three sets of bores 72, 102, and 106 may be distributed symmetrically around an axis 110 of cap 100. However, the bores are not necessarily distributed symmetrically around axis 110.

Each through longitudinal bore 102 terminates in a an opening 114 in the outer surface of wall 74, and a transparent window 116 is placed in the opening. A fiber optic 118 is inserted into each of the through bores. There is a respective opening 120, in the outer surface of wall 74, to each blind bore 106, and a transparent window 124 is placed in each opening 120. A fiber optic 128 is inserted into each of the blind bores. Windows 116 and 124 act as seals preventing fluid external to the outer surface of cap 70 from penetrating into the bores containing the fiber optics. Windows 116 and 124 may be formed by filling openings 114 and 120 with an optically transparent glue or epoxy. In some embodiments the material of the windows may be filled with a scattering agent to diffuse light passing through the windows. Alternatively, the windows may be formed from an optical quality flat or lensed material, and may be secured to their openings with glue.

In a disclosed embodiment each fiber optic 118 or each fiber optic 128 is a single fiber optic, typically having a diameter of approximately 175 µm. In an alternative disclosed embodiment each fiber optic 118 or each fiber optic 128 comprises a bundle of substantially similar fiber optics, typically having a bundle diameter also of approximately 175 µm. Implementing the fiber optics as bundles increases the flexibility of cap 100 with respect to more proximal regions of catheter 22.

Such an increase in flexibility is advantageous if cap 100 is connected to the more proximal regions of the catheter by a spring whose deflections are measured for the purpose of measuring a force on the cap, since the increased flexibility means there is little or no change in the spring deflection for a given force. A spring which may be used to join the cap 100 to the more proximal regions of the catheter is described in U. S. Patent Application 12/627,327, to Beeckler et al., published as US2011/0130648.

Optical module 40 is configured to be able to provide optical radiation to any one of fiber optics 118 and 128, for transmission from any of the associated windows 116 and 124 so as to irradiate tissue in proximity to cap 100. Simultaneously, the module is able to acquire, via any or all of the windows, radiation returning from the irradiated tissue.

The array of windows 116 and 124, and their associated fiber optics, enables embodiments of the present invention to employ a number of different methods, using optical radiation, for determining characteristics of the irradiated tissue, as well as the proximity of cap 100, or a region of the cap, with respect to the tissue. By way of example three such methods are described below, but those having ordinary skill in the art will be aware of other methods.

A first method detects contact of any one of windows 116 or 124, and consequently of the catheter, with tissue. Optical radiation, of a known intensity, is transmitted through each fiber optic, so as to exit from the optic's window. The intensity of the radiation returning to the window is measured while cap 100 is not in contact with tissue, typically while the cap is in the blood of heart 24. Optical monitor 40 may use these intensities as reference values of the optical radiation. For any given window, a change in the value from the window's reference value, as measured by the module, may be taken to indicate that the window is in contact with tissue.

A second method measures characteristics of tissue being irradiated by the optical radiation. As illustrated in Fig. 5E, for all six windows 116, 124 there are a total of 21 different paths, comprising 6 paths 150 where radiation from a given window returns to that window, and 15 paths 160 where radiation from a given window returns to a different window. The change of optical radiation for a given path or group of paths depends on characteristics of tissue in the path or group of paths, so that measurements of the change in all of the paths provide information related to characteristics of the tissue in proximity to cap 100.

For example, the change in all of the paths may be measured by sequentially transmitting, in a time multiplexed manner, optical radiation from each of windows 116 and 124, and measuring the returning radiation. A first transmission from a first window in such a sequence provides values for five paths 160 plus a return path 150 to the first window, a second transmission from a second window provides values for four new paths 160 plus a return path 150 to the second window, ... a fifth transmission from a fifth window provides values for two new paths (a path 160 to the sixth window, and a return path 150 to the fifth window). A sixth and final transmission from a sixth window provides one return path 150 through the sixth window.

Optical module 40 may measure the changes of all the paths, and, typically using a calibration procedure, may derive from the changes optical characteristics of tissue within the paths. Such characteristics may include an overall level of ablation of tissue, or an amount and/or type of necrotic tissue, in the paths.

A third method uses changes of levels of optical radiation returning to windows 116 and/or 124, such as are described in the two methods, to make an estimate of the wall thickness of tissue being illuminated by the optical radiation.

Although a number of particular implementation examples have been shown and described above, alternative implementations of the principles embodied in these examples will be apparent to those skilled in the art after reading the foregoing description and are considered to be within the scope of the present invention.

For example, in one embodiment, temperature sensors 48 may not be installed in wall 74, and only fiber optics 118 are incorporated into the wall. Such an embodiment enables determination of tissue contact with the cap, and/or characterization of the tissue in proximity to the cap, by methods described above.

## Claims

1. Apparatus (22), comprising:
an insertion tube (42) having a distal end (30) configured for insertion into proximity with tissue in a body of a patient and containing a lumen (54) comprising an electrical conductor for conveying electrical energy to the tissue;
a conductive cap (100) attached to the distal end of the insertion tube (42) and coupled electrically to the electrical conductor, wherein the conductive cap (100) has an outer surface;
a multiplicity of optical fibers (118, 128) contained within the insertion tube (42), each fiber terminating in proximity to the outer surface of the cap (100), **characterized in that** each fiber is configured to convey optical radiation to and from the tissue while the electrical energy is being conveyed to the tissue and **in that** the apparatus further comprises an optical module (40) configured to determine contact of the conductive cap (100) with the tissue in response to measuring a first level of the optical radiation conveyed via a given optical fiber (118; 128) to the tissue and a second level of the optical radiation conveyed via the given optical fiber (118; 128) from the tissue.

2. The apparatus (22) according to claim 1, and comprising a plurality of temperature sensors (48), which are mounted within the conductive cap (100) in thermal communication with the outer surface.

3. The apparatus (22) according to claim 1, wherein the outer surface is penetrated by multiple apertures, and wherein the conductive cap (100) defines an inner cavity (76) in fluid communication with the lumen (54) of the insertion tube (42) so as to permit irrigation fluid from the lumen (54) to flow out of the cap (100) through the apertures.

4. The apparatus (22) according to claim 1, wherein the cap (100) comprises a side wall (74) having a multiplicity of longitudinal bores (102, 106) therein, and wherein the fiber optics (118, 128) are inserted into the bores (102, 106) .

5. The apparatus (22) according to claim 4, and comprising a multiplicity of windows (116, 124), transparent to the optical radiation, located in the outer surface of the cap (100) and connected to respective longitudinal bores (102, 106) so as to seal the bores (102, 106) from penetration of fluid into the bores (102, 106).

6. The apparatus (22) according to claim 5, wherein at least one of the windows (116, 124) is formed from at least one of a transparent epoxy and a glue.

7. The apparatus (22) according to claim 6, and comprising a scattering agent mixed with at least one of the windows (116, 124).

8. The apparatus (22) according to claim 5, wherein at least one of the windows (116, 124) is formed from at least one of optical grade flat material and optical grade lensed material.

9. The apparatus (22) according to claim 1, wherein a given optical fiber (118; 128) selected from the multiplicity of optical fibers (118, 128) comprises a single optical fiber (118; 128).

10. The apparatus (22) according to claim 1, wherein a given optical fiber (118; 128) selected from the multiplicity of optical fibers (118, 128) comprises an optical fiber (118, 128) bundle.

11. The apparatus (22) according to claim 1, and comprising an optical module (40) configured to determine a characteristic of the tissue in response to measuring a first level of the optical radiation conveyed via a first optical fiber (118, 128) to the tissue and a second level of the optical radiation conveyed via a second optical fiber (118, 128) from the tissue.

12. The apparatus (22) according to claim 11, wherein the characteristic comprises a wall thickness of the tissue.

13. The apparatus (22) according to claim 1, and comprising a power generator (34), coupled to provide the electrical energy to the conductive cap (100) so as to ablate the tissue, and an optical module (40) configured to determine a change in a level of the optical radiation while the tissue is ablating.

## Patentansprüche

1. Vorrichtung (22), umfassend:
ein Einführungsrohr (42) mit einem distalen Ende (30), das zur Einführung in die Nähe zu Gewebe in einem Körper eines Patienten ausgelegt ist und ein Lumen (54) enthält, das einen elektrischen Leiter zur Übertragung elektrischer Energie an das Gewebe umfasst;
eine leitfähige Kappe (100), die am distalen Ende des Einführungsrohrs (42) befestigt ist und elektrisch mit dem elektrischen Leiter verbunden ist, wobei die leitfähige Kappe (100) eine Außenfläche aufweist;
eine Vielzahl von optischen Fasern (118, 128), die im Einführungsrohr (42) enthalten sind, wobei jede Faser in der Nähe der Außenfläche der Kappe (100) endet,
**dadurch gekennzeichnet, dass** jede Faser zur Übertragung optischer Strahlung zum und vom Gewebe ausgelegt ist, während die elektrische Energie zum Gewebe übertragen wird,
und dass die Vorrichtung ferner ein optisches Modul (40) umfasst, das zum Bestimmen eines Kontakts der leitfähigen Kappe (100) mit dem Gewebe als Reaktion auf eine Messung eines ersten Niveaus der optischen Strahlung, die über eine gegebene optische Faser (118; 128) an das Gewebe übertragen wird, und eines zweiten Niveaus der optischen Strahlung, die über die gegebene optische Faser (118; 128) vom Gewebe übertragen wird, ausgelegt ist.

2. Vorrichtung (22) nach Anspruch 1, und umfassend eine Vielzahl von Temperatursensoren (48), die in der leitfähigen Kappe (100) in Wärmeverbindung mit der Außenfläche angebracht sind.

3. Vorrichtung (22) nach Anspruch 1, wobei die Außenfläche von mehreren Öffnungen penetriert wird und wobei die leitfähige Kappe (100) einen inneren Hohlraum (76) in Fluidverbindung mit dem Lumen (54) des Einführungsrohrs (42) definiert, damit Spülflüssigkeit aus dem Lumen (54) durch die Öffnungen aus der Kappe (100) fließen kann.

4. Vorrichtung (22) nach Anspruch 1, wobei die Kappe (100) eine Seitenwand (74) mit einer Vielzahl von Längsbohrungen (102, 106) darin umfasst und wobei die Faseroptiken (118, 128) in die Bohrungen (102, 106) eingeführt werden.

5. Vorrichtung (22) nach Anspruch 4, und umfassend eine Vielzahl von Fenstern (116, 124), die gegenüber der optischen Strahlung transparent sind, in der Außenfläche der Kappe (100) angeordnet sind und mit jeweiligen Längsbohrungen (102, 106) verbunden sind, um die Bohrungen (102, 106) gegen Eindringen von Fluid in die Bohrungen (102, 106) abzudichten.

6. Vorrichtung (22) nach Anspruch 5, wobei zumindest eines der Fenster (116, 124) aus zumindest einem eines transparenten Epoxys und eines Klebstoffs gebildet wird.

7. Vorrichtung (22) nach Anspruch 6, und umfassend ein Streuungsmittel, das mit zumindest einem der Fenster (116, 124) gemischt ist.

8. Vorrichtung (22) nach Anspruch 5, wobei zumindest eines der Fenster (116, 124) aus zumindest einem eines optischen Flachmaterials und eines optischen, mit Linse versehenen Materials gebildet wird.

9. Vorrichtung (22) nach Anspruch 1, wobei eine gegebene optische Faser (118; 128), die aus einer Vielzahl von optischen Fasern (118; 128) ausgewählt ist, eine einzelne optische Faser (118; 128) umfasst.

10. Vorrichtung (22) nach Anspruch 1, wobei eine gegebene optische Faser (118; 128), die aus der Vielzahl von optischen Fasern (118; 128) ausgewählt ist, ein optisches Faserbündel (118, 128) umfasst.

11. Vorrichtung (22) nach Anspruch 1, und umfassend ein optisches Modul (40), das zur Bestimmung einer Eigenschaft des Gewebes als Reaktion auf eine Messung eines ersten Niveaus der optischen Strahlung, die über eine erste optische Faser (118, 128) zum Gewebe übertragen wird, und eines zweiten Niveaus der optischen Strahlung, die über eine zweite optische Faser (118, 128) vom Gewebe übertragen wird, ausgelegt ist.

12. Vorrichtung (22) nach Anspruch 11, wobei die Eigenschaft eine Wandstärke des Gewebes umfasst.

13. Vorrichtung (22) nach Anspruch 1, und umfassend einen Stromgenerator (34), der zur Bereitstellung der elektrischen Energie an die leitfähige Kappe (100) zum Ablatieren von Gewebe ausgelegt ist, und ein optisches Modul (40), das zur Bestimmung einer Veränderung in einem Niveau der optischen Strahlung während der Ablation des Gewebes ausgelegt ist.

## Revendications

1. Appareil (22), comprenant :
un tube d'insertion (42) ayant une extrémité distale (30) configurée pour l'insertion à proximité d'un tissu dans le corps d'un patient et contenant une lumière (54) comprenant un conducteur électrique pour transporter de l'énergie électrique au tissu ;
un capuchon conducteur (100) fixé à l'extrémité distale du tube d'insertion (42) et couplé électriquement au conducteur électrique, le capuchon conducteur (100) ayant une surface externe ;
une multiplicité de fibres optiques (118, 128) contenues dans le tube d'insertion (42), chaque fibre se terminant à proximité de la surface extérieure du capuchon (100), **caractérisé en ce que** chaque fibre est configurée pour transporter le rayonnement optique vers et depuis le tissu pendant que l'énergie électrique est transportée au tissu et **en ce que** l'appareil comprend en outre
un module optique (40) configuré pour déterminer un contact du capuchon conducteur (100) avec le tissu en réponse à une mesure d'un premier niveau du rayonnement optique transporté via une fibre optique donnée (118 ; 128) au tissu et d'un deuxième niveau du rayonnement optique transporté via la fibre optique donnée (118 ; 128) à partir du tissu.

2. Appareil (22) selon la revendication 1, et comprenant une pluralité de capteurs de température (48), qui sont montés dans le capuchon conducteur (100) en communication thermique avec la surface extérieure.

3. Appareil (22) selon la revendication 1, la surface extérieure étant traversée par de multiples ouvertures, et le capuchon conducteur (100) définissant une cavité intérieure (76) en communication fluidique avec la lumière (54) du tube d'insertion (42) de manière à permettre à du fluide d'irrigation de la lumière (54) de sortir du capuchon (100) par les ouvertures.

4. Appareil (22) selon la revendication 1, le capuchon (100) comprenant une paroi latérale (74) ayant une multiplicité d'alésages longitudinaux (102, 106), et les fibres optiques (118, 128) étant insérées dans les alésages (102, 106).

5. Appareil (22) selon la revendication 4, et comprenant une multiplicité de fenêtres (116, 124), transparentes au rayonnement optique, situées dans la surface extérieure du capuchon (100) et reliées à des trous longitudinaux respectifs (102, 106) de manière à sceller les trous (102, 106) de manière à empêcher la pénétration du fluide dans les trous (102, 106).

6. Appareil (22) selon la revendication 5, au moins l'une des fenêtres (116, 124) étant formée d'au moins une résine époxyde transparente et d'une colle.

7. Appareil (22) selon la revendication 6, et comprenant un agent dispersant mélangé avec au moins une des fenêtres (116, 124).

8. Appareil (22) selon la revendication 5, au moins une des fenêtres (116, 124) étant formée d'au moins un matériau plat de qualité optique et d'un matériau de lentille de qualité optique.

9. Appareil (22) selon la revendication 1, une fibre optique donnée (118 ; 128) choisie parmi la multiplicité de fibres optiques (118, 128) comprenant une fibre optique unique (118 ; 128).

10. Appareil (22) selon la revendication 1, une fibre optique donnée (118 ; 128) choisie parmi la multiplicité de fibres optiques (118, 128) comprenant un faisceau de fibres optiques (118, 128).

11. Appareil (22) selon la revendication 1, et comprenant un module optique (40) configuré pour déterminer une caractéristique du tissu en réponse à la mesure d'un premier niveau du rayonnement optique transporté via une première fibre optique (118, 128) vers le tissu et d'un second niveau du rayonnement optique transporté via une seconde fibre optique (118, 128) à partir du tissu.

12. Appareil (22) selon la revendication 11, la caractéristique comprenant une épaisseur de paroi du tissu.

13. Appareil (22) selon la revendication 1, et comprenant un générateur de puissance (34), couplé pour fournir l'énergie électrique au capuchon conducteur (100) de manière à éliminer le tissu, et un module optique (40) configuré pour déterminer un changement dans un niveau du rayonnement optique lorsque le tissu est éliminé.
